# FASCICULE DE BREVET EUROPEEN

(11) **EP 1 602 344 B1**
(45) Date de publication et mention de la délivrance du brevet: **01.08.2007**
(21) Numéro de dépôt: 04291404.4
(22) Date de dépôt: 04.06.2004
(51) Int. Cl.: A61F 2/84, A61M 25/00

(54) **Dispositif de mise en place d'une endoprothèse auto-expansible**
Katheter zum Einbringen eines selbstexpandierbaren Stents
Catheter for deployment of a self-expandible stent

(43) Date de publication de la demande: 07.12.2005
(73) Titulaire: MS Techniques, 54340 Pompey (FR)
(72) Inventeur: Malher, Etienne, 54000 Nancy (FR)
(74) Mandataire: Lagrange, Jacques Etienne M.M.

(56) Documents cités:
- EP-A- 0 699 451
- WO-A-99/47075
- US-A- 6 030 413

## Description

La présente invention est relative à un dispositif pour la mise en place d'une endoprothèse auto-expansible.

Les endoprothèses sont des implants médicaux de forme générale tubulaire en métal ou en matériau polymère destinés à maintenir ouvert un canal qui a tendance à se fermer sous l'effet d'une pathologie (tumeur, plaque d'athérome, etc...). Ce canal peut être par exemple une artère, une veine, une voie respiratoire, une voie urinaire, un canal biliaire.

Ces implants sont introduits dans un état contracté afin de pouvoir être amenés jusqu'au lieu d'implantation de façon peu traumatique. Ils sont ensuite dilatés à leur dimension définitive soit par déformation plastique soit par leur propre retour élastique. Les implants dilatés à leur dimension définitive par leur propre retour élastique constituent des endoprothèses, dites auto-expansibles, décrites par exemple par WALLSTEN Hans Ivar dans le brevet FR 2 525 896.

Ces endoprothèses sont mises en place à l'aide d'un dispositif constitué principalement :
- d'une gaine extérieure qui permet de maintenir l'endoprothèse dans sa configuration comprimée. Cette gaine s'étend de son extrémité proximale qui reste en dehors du corps, à son extrémité distale au niveau de laquelle se trouve l'endoprothèse.
- d'un mandrin intérieur d'une longueur adaptée à celle de la gaine, coulissant dans celle-ci et permettant par un mouvement de translation relatif de pousser l'endoprothèse hors de la gaine. Ce mandrin est solidarisé temporairement à l'endoprothèse soit par une fine couche de matériau souple qui adhère au mandrin et dans laquelle vient s'incruster l'endoprothèse, configuration décrite par BURTON John H. et coll. dans le brevet EP 0 408 245, soit par une butée qui vient au contact de l'endoprothèse telle que décrite par WILSON David J. et coll. dans le brevet EP 1 025 813, soit par d'autres dispositifs mécaniques comme par exemple celui décrit par CRYER Brett W. dans le brevet WO 02/41 805. Ce mandrin traverse l'endoprothèse et se termine par une extrémité souple et arrondie qui s'ajuste à l'extrémité de la gaine et permet une progression de l'ensemble pendant l'introduction sans risquer d'endommager les tissus.

Afin de faciliter le cheminement de l'instrument lors de sa progression vers la zone à traiter, un fil-guide est préalablement introduit jusqu'à cette zone.

On connaît deux configurations :
- la configuration dite coaxiale dans laquelle le mandrin est percé de part en part de façon à permettre le passage du fil-guide sur toute sa longueur.
- la configuration dite « échange rapide », décrite par HEALY Stephen R. et COLL. dans le brevet EP 1 095 634. Dans cette configuration, le mandrin présente un percement entre l'extrémité distale et une ouverture latérale située à une certaine distance de cette extrémité. Au lieu de traverser tout le dispositif, le fil-guide ne traverse que cette courte distance.

Pour mettre en place la prothèse, le praticien introduit tout d'abord le fil-guide dans le canal à travers et au-delà de la zone à traiter, puis il introduit l'extrémité du fil-guide restée à l'extérieur du corps dans la partie distale du mandrin. Il le fait ensuite glisser jusqu'à le faire ressortir soit par l'extrémité proximale - configuration coaxiale - soit par l'ouverture latérale - « échange rapide » - puis fait cheminer le dispositif le long de ce guide jusqu'à ce que l'extrémité distale soit au niveau de la zone à traiter de façon à positionner l'endoprothèse en face de la zone à traiter.

Il effectue ensuite un mouvement de translation relatif mandrin/gaine qui permet de chasser hors de la gaine l'endoprothèse. Pendant cette translation, il s'efforce de maintenir le mandrin fixe par rapport au canal tout en reculant la gaine afin que l'endoprothèse reste bien en face de la zone à traiter. Quand l'endoprothèse est entièrement larguée, il peut retirer le dispositif.

Le positionnement correct de l'endoprothèse est un élément indispensable pour la réussite de l'opération. Pour y parvenir, il faut absolument contrôler la stabilité du mandrin dans sa partie distale tout au long du largage.

Pour obtenir ce résultat, il a été proposé des poignées de manoeuvre qui réalisent un mouvement de recule de la gaine tout en maintenant le mandrin fixe par rapport à la main de l'opérateur. Un dispositif de ce genre est décrit par SULLIVAN Roy dans le brevet US 5 968 052.

La principale insuffisance de ce type d'instrument est qu'il ne peut garantir un contrôle correct du mouvement de la partie distale, en particulier dans le cas où l'instrument suit plusieurs courbes à l'intérieur des canaux qui le mènent jusqu'à la zone à traiter. Le comportement souhaité de l'instrument est tel que le mandrin reste fixe dans sa partie distale comme dans sa partie proximale et la gaine recule dans sa partie distale comme dans sa partie proximale. Mais, un comportement différent peut être provoqué par exemple par un frottement important entre la face externe de la gaine et la paroi du canal à traiter. Dans ce cas, le mouvement de la gaine est correcte au niveau proximal, mais celle-ci demeure immobile par rapport à la zone à traiter à son extrémité distale, de telle sorte que la compensation du mouvement se fait par une modification des courbes de l'instrument et c'est le mandrin qui, sortant de la gaine, se déplace par rapport au canal à traiter.

Ce phénomène est encore aggravé dans le cas du traitement d'organe en mouvement comme par exemple les artères coronaires.

On notera que, souvent, la mise en place de l'endoprothèse est précédée d'une angioplastie destinée à dilater le canal à traiter. Cette angioplastie est réalisée à l'aide d'un cathéter comportant un ballonnet pour angioplastie constitué d'un matériau résistant et peu élastique, de façon à pouvoir être gonflé jusqu'à présenter un diamètre déterminé, sous une pression élevée et pouvant dépasser 10 bars.

Afin de ne pas avoir à utiliser deux cathéters successifs, il a été proposé, notamment dans la demande de brevet EP 0 699 451, un cathéter intégré comportant à son extrémité distale un ballonnet pour angioplastie et un moyen de largage d'une endoprothèse auto-expansible. Mais un tel cathéter ne résout pas le problème du positionnement au moment de la mise en place de l'endoprothèse.

Pendant le largage, le ballonnet de dilatation se trouve en aval de la zone traitée, donc dans un canal sain et sensiblement plus étroit. De par la construction du ballonnet, un gonflage, même à très faible pression, provoque une dilatation du canal, ce qui est un facteur très défavorable pour le phénomène de re-sténose. Il est indiqué dans la demande de brevet citée plus haut que le ballonnet reste dégonflé pendant le largage.

Un cathéter comportant un ballonnet du type ballonnet pour angioplastie ont également été proposés, notamment dans le brevet US 6,030,413 pour mettre en place des greffons maintenus par des agrafes. Mais un tel cathéter n'est pas adapté à la mise en place d'endoprothèses auto-expansibles.

Le but de l'invention est de proposer un dispositif de largage d'une endoprothèse auto-expansible dont le mandrin peut être immobilisé dans sa partie distale à l'endroit du canal où le praticien aura choisi d'effectuer le largage, cet élément pouvant être activé et désactivé au souhait du praticien.

A cet effet, l'invention a pour objet un dispositif de mise en place d'une endoprothèse auto-expansible, le dispositif s'étendant entre une extrémité proximale et une extrémité distale et comprenant :
- une gaine extérieure capable de maintenir une endoprothèse en contraction radiale,
- un mandrin s'étendant axialement à l'intérieur de la gaine extérieure, mobile axialement par rapport à la gaine extérieure et comprenant à son extrémité distale une chambre pouvant recevoir une endoprothèse en contraction radiale, ladite chambre étant délimitée à son extrémité distale par une pointe atraumatique pouvant obstruer ladite gaine extérieure, caractérisé en ce que la pointe atraumatique est en matériau élastomère et comporte une chambre gonflable à paroi mince pouvant être gonflée par une pression insuffisante pour élargir un canal destiné à recevoir la prothèse, dans laquelle débouche une lumière s'étendant de l'extrémité distale à l'extrémité proximale du mandrin pour le passage d'un fluide de gonflage.

La chambre gonflable de la pointe atraumatique peut être gonflée par une pression inférieure à 1 bar.

Le matériau élastomère dont est constituée la pointe atraumatique est par exemple un élastomère pris parmi le silicone, le caoutchouc naturel, le polyuréthane et le polyéther-bloc-amide.

Le mandrin peut comprendre une deuxième lumière s'étendant de l'extrémité distale à l'extrémité proximale pour le passage d'un fil guide, ladite deuxième lumière se prolongeant dans l'axe de la pointe atraumatique.

La lumière du mandrin pour la circulation d'un fluide de gonflage peut avoir un diamètre suffisant pour permettre simultanément le passage d'un fil guide et la circulation d'un fluide de gonflage. La pointe atraumatique peut alors comporter un trou axial pour le fil guide, dont la paroi interne comporte au moins un bourrelet pour assurer l'étanchéité du contact avec le fil guide.

Le dispositif peut comporter une gaine coaxiale, située à l'intérieur de la lumière du mandrin permettant la circulation d'un fluide de gonflage sans obstruer ladite lumière, s'étendant depuis l'extrémité distale du mandrin et débouchant à l'extérieur du dispositif par une ouverture située entre l'extrémité distale et l'extrémité proximale, et permettant le passage d'un file guide, ladite gaine se prolongeant dans l'axe de la pointe atraumatique de façon à assurer l'étanchéité avec le mandrin et à laisser libre le passage du fil guide.

L'invention a également pour objet un procédé pour mettre en place une endoprothèse auto-expansible dans un canal à l'aide d'un dispositif selon l'invention.

Selon le procédé :
- on introduit le dispositif dans le canal jusqu'à la zone à traiter ;
- on introduit un volume défini de liquide de gonflable dans la chambre de la pointe atraumatique de façon à former un ballonnet qui immobilise la pointe du dispositif par rapport au canal ;
- on libère l'endoprothèse en reculant la gaine extérieure ;
- on dégonfle le ballonnet et on retire le dispositif.

L'invention va maintenant être décrite plus en détails mais de façon limitative en regard des figures annexées, dans lesquelles :
- les figures 1A, 1B, 1C, 1D, 1E représentent en coupe longitudinale l'extrémité distale d'un dispositif de largage d'une endoprothèse auto-expansible à l'intérieur d'un canal destiné à recevoir l'endoprothèse, aux cinq étapes principales de largage de l'endoprothèse ;
- la figure 2 est une coupe agrandie de l'extrémité distale d'un premier mode de réalisation d'un dispositif de largage d'une endoprothèse ;
- la figure 3 est une vue en coupe agrandie de l'extrémité distale d'un second mode de réalisation d'un dispositif de largage d'une endoprothèse ;
- la figure 4 est une vue en coupe agrandie de l'extrémité distale d'un troisième mode de réalisation d'un dispositif de largage d'une endoprothèse auto-expansible.

Le dispositif de mise en place d'une endoprothèse auto-expansible est constitué :
- d'une gaine extérieure 1 qui permet de maintenir une endoprothèse 2 dans sa configuration comprimée. Cette gaine s'étend de son extrémité proximale qui reste en dehors du corps, à son extrémité distale 3, au niveau de laquelle se trouve l'endoprothèse 21.
- d'un mandarin intérieur 4 d'une longueur adaptée à celle de la gaine 1, s'étendant axialement à l'intérieur de la gaine, coulissant dans celle-ci et permettant par un mouvement de translation relatif de pousser l'endroprothèse 21 hors de la gaine. Ce mandrin est solidarisé temporairement à l'endoprothèse par une butée 5 qui vient au contact de l'endoprothèse telle que décrite par WILSON David J. et Coll. dans le brevet EP 1 025 813.

Ce mandrin traverse l'endoprothèse et se termine par une pointe atraumatique 6 qui s'ajuste à l'extrémité de la gaine 1 en délimitant avec la butée 5 une chambre 7 pouvant recevoir une endoprothèse 21. Cette pointe atraumatique permet une progression de l'ensemble pendant l'introduction sans risquer d'endommager les tissus.

Afin de faciliter le cheminement du dispositif de mise en place d'une endoprothèse lors de sa progression vers la zone à traiter, un fil-guide 8 est préalablement introduit jusqu'à cette zone et, au moins l'extrémité distale du dispositif de mise en place d'une endoprothèse peut coulisser le long de ce fil.

Dans un premier mode de réalisation du fil-guide, le mandrin comporte une lumière 9 ou 9'A s'étendant sur toute sa longueur de façon à permettre le passage du fil-guide sur toute la longueur du mandrin.

Dans un deuxième mode de réalisation du fil-guide, dit à « échange rapide », décrit par HEALY Stephen R. et Coll. dans le brevet EP 1 095 634, le mandrin comporte une lumière 9B s'étendant depuis l'extrémité distale du dispositif de mise en place d'une endoprothèse, jusqu'à une ouverture latérale 14 prévue dans la paroi du dispositif de mise en place d'une endoprothèse, et débouchant ainsi à l'extérieur entre l'extrémité distale et l'extrémité proximale du dispositif. Dans le mode de réalisation, le fil-guide ne traverse qu'une courte longueur du dispositif de mise en place d'une prothèse, ce qui facilite les manipulations du dispositif.

La pointe atraumatique 6 est réalisée en un matériau souple et élastique du type élastomère, et par exemple du type élastomère réticulé tel que le silicone ou le caoutchouc naturel, ou du type élastomère thermoplastique tel que le polyuréthane ou le polyétherblocamide.

Cette pointe atraumatique 6 comporte une chambre a paroi mince 10 qui peut être gonflée sous une pression modérée pour former un ballonnet. Ce ballonnet est comparable au ballonnet d'une sonde de Fogarty, c'est-à-dire qu'il n'est pas destiné, et ne peut pas servir à dilater le canal dans lequel il est placé. Ce ballonnet est destiné à être gonflé par un liquide tel que du sérum physiologique ou du liquide de contraste, amené par une lumière s'étendant sur toute la longueur du mandrin 4, et délivré par une seringue à limite de volume (non représentée comme en elle-même) de façon à limiter l'expansion du ballonnet.

Avec un tel dispositif, la pression de gonflage du ballonnet est insuffisante pour dilater un canal comportant un athérome. Le volume de liquide injecté provoque la dilatation du ballonnet jusqu'au contact avec la parois du canal, puis l'excédent de volume est absorbé par l'allongement du ballonnet. Cette pression peut être inférieure à 1 bars.

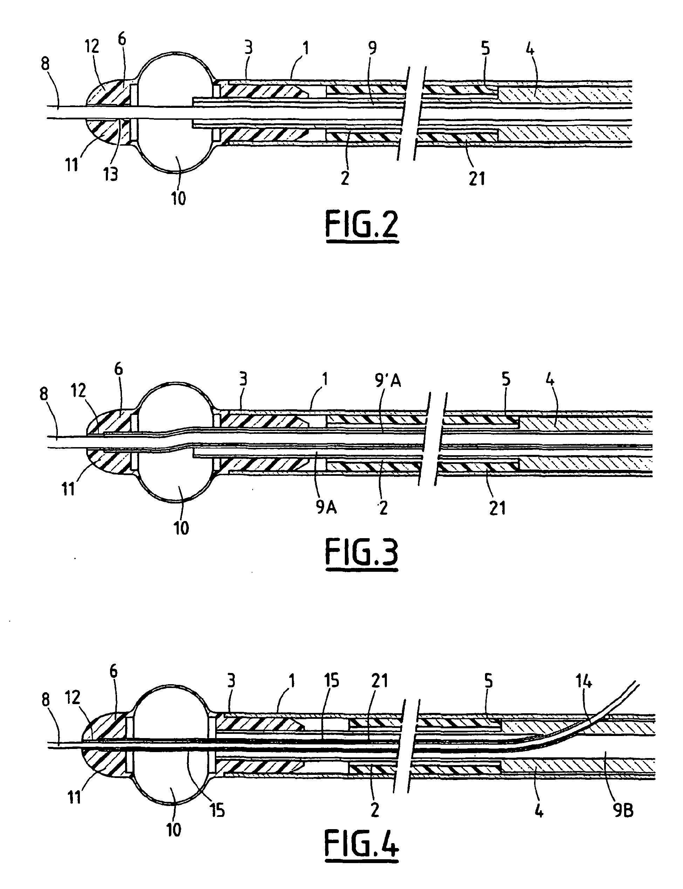

Plusieurs modes de réalisation de la pointe atraumatique et de sa liaison avec le mandrin sont possibles. Trois modes de réalisation sont représentés aux figures 2, 3 et 4.

Dans un premier mode de réalisation, représenté à la figure 2, le mandrin comporte une seule lumière 9 de diamètre suffisant pour laisser passer à la fois le fil-guide 8 et le liquide de gonflage. Cette lumière 9 débouche dans la chambre 10 destiné à former un ballonnet. Le fil-guide 8 traverse la chambre 10 puis l'extrémité 11 de la pointe atraumatique par un trou axial 12 qui comporte un bourrelet intérieur 13 destiné à assurer l'étanchéité du contact avec le fil-guide.

Dans un deuxième mode de réalisation représenté à la figure 3, le mandrin 4 se prolonge jusqu'à l'extrémité 11 de la pointe atraumatique 6.

Il comporte une première lumière axiale 9'A destinée à recevoir le fil-guide 8 débouchant dans le trou axial 12 de l'extrémité 11 de la pointe atraumatique 6, et une deuxième lumière 9A parallèle à la première, destinée à recevoir le liquide de gonflage et débouchant dans la chambre 10 destinée à former un ballonnement.

Dans un troisième mode de réalisation, représenté à la figure 4, correspondant à un dispositif à « échange-rapide », le mandrin 4 comporte une lumière 9B destinée à recevoir le liquide de gonflage, et dans sa partie distale à recevoir en outre le fil-guide 8 qui, du côté proximal, sort du mandrin par une ouverture latérale 14. Le fil-guide traverse l'extrémité 11 de la pointe atraumatique 6 par un trou axial 12 et l'étanchéité est assurée par une gaine 15 dans laquelle le fil-guide 8 peut glisser. Cette gaine 15 s'étend de l'extrémité 11 de la pointe atraumatique 6 jusqu'à l'ouverture latérale 14 prévue dans le mandrin pour permettre au fil-guide de sortir latéralement.

Dans les modes de réalisation qui ont été décrits précédemment, l'endophothèse est maintenue en place sur le mandrin par l'intermédiaire d'une butée 5. Mais d'autres modes de réalisation, faisant partie de l'invention, sont possibles. Il s'agit par exemple, de dispositif mécanique tel que celui décrit par CRYER Brett. W. dans la demande de brevet WO 02/41 805, ou d'une fine couche de matériau souple qui adhère au mandrin et dans laquelle vient s'incruster l'endoprothèse, configuration décrite par BURTON John H. et Coll. dans le brevet EP 0 408 245.

Dans tous les cas, la gaine extérieure et le mandrin sont reliés, à leur extrémités proximales, à des poignées, connues en elles-mêmes, permettant d'assurer un mouvement de translation relatif du mandrin par rapport à la gaine.

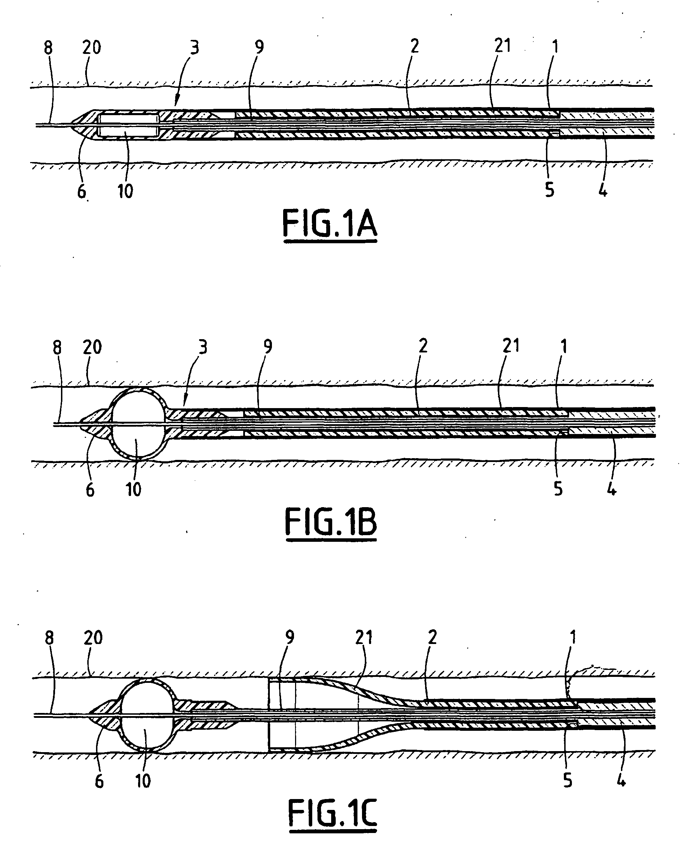

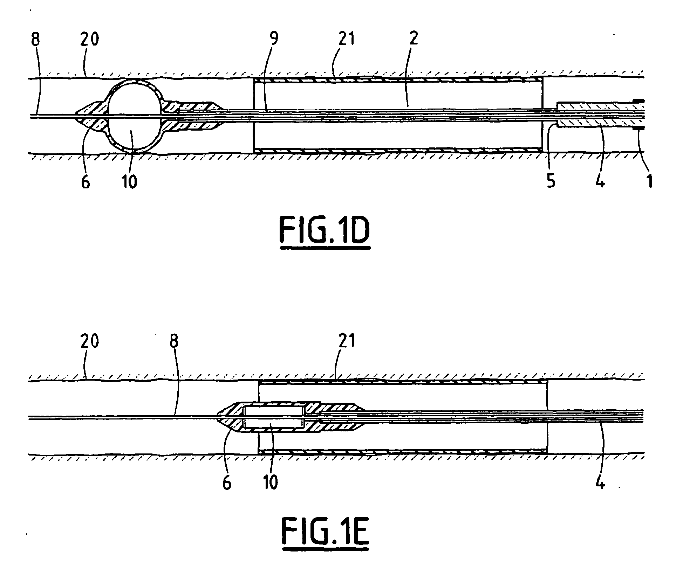

Les séquences d'utilisation du dispositif pour mettre une endoprothèse dans un canal sont représentées aux figures 1 A à 1 E.

Etape A : Le dispositif est avancé dans le canal 20 jusqu'à la zone à traiter.

Etape B : Le ballonnement 10 est gonflé, ce qui immobilise l'extrémité du mandrin 4.

Etape C : Le praticien exerce un mouvement de recul de la gaine 1 dans sa partie distale en raison de l'immobilisation de la partie distale du mandrin. L'endoprothèse 21 commence à se déployer.

Etape D : La gaine est entièrement reculée, l'endoprothèse 21 est entièrement libérée.

Etape E : Le ballonnet 10 est dégonflé, ce qui libère le dispositif qui est alors retiré du canal à traiter.

## Revendications

1. Dispositif de mise en place d'une endoprothèse auto-expansible, s'étendant entre une extrémité proximale et une extrémité distale et comprenant :
- une gaine extérieure (1) capable de maintenir une endoprothèse (21) en contraction radiale,
- un mandrin (4) s'étendant axialement à l'intérieur de la gaine extérieure (1), mobile axialement par rapport à la gaine extérieure et comprenant à son extrémité distale (3) une chambre (2) pouvant recevoir une endoprothèse (21) en contraction radiale, ladite chambre étant délimitée à son extrémité distale par une pointe atraumatique (6) pouvant obstruer ladite gaine extérieure, la pointe atraumatique (6) est en matériau élastomère et comporte une chambre gonflable (10), et une lumière (9, 9A, 9B) s'étendant de l'extrémité distale à l'extrémité proximale du mandrin (4) pour le passage d'un fluide de gonflage débouche dans la chambre gonflable (10),
**caractérisé en ce que** la chambre gonflable (10) de la pointe atraumatique (6) est à paroi mince et peut être gonflée par une pression inférieure à 1 bar insuffisante pour élargir un canal destiné à recevoir la prothèse, afin de former un ballonnet qui immobilise la pointe du mandrin par rapport au canal.

2. Dispositif selon la revendication 1, **caractérisé en ce que** le matériau élastomère dont est constituée la pointe atraumatique (6) est un élastomère pris parmi le silicone, le caoutchouc naturel, le polyuréthane et le polyéther-bloc-amide.

3. Dispositif selon l'une quelconque des revendications 1 à 2, **caractérisé en ce que** le mandrin (4) comprend une deuxième lumière (9'A) s'étendant de l'extrémité distale à l'extrémité proximale pour le passage d'un fil guide (8), ladite deuxième lumière se prolongeant dans l'axe de la pointe atraumatique.

4. Dispositif selon l'une quelconque des revendications 1 à 2, **caractérisé en ce que** la lumière (9) du mandrin (4) pour la circulation d'un fluide de gonflage a un diamètre suffisant pour permettre simultanément le passage d'un fil guide (8) et la circulation d'un fluide de gonflage.

5. Dispositif selon la revendication 4, **caractérisé en ce que** la pointe atraumatique (6) comporte un trou axial (12) pour le passage d'un fil guide (8), dont la paroi interne comporte au moins un bourrelet (13) pour assurer l'étanchéité du contact avec le fil guide (8).

6. Dispositif selon l'une quelconque des revendications 1 à 2, **caractérisé en ce qu**'il comporte, en outre, une gaine coaxiale (15), située à l'intérieur de la lumière (9B) du mandrin (4) permettant la circulation d'un fluide de gonflage sans obstruer ladite lumière, s'étendant depuis l'extrémité distale du mandrin et débouchant à l'extérieur du dispositif par une ouverture (14) située entre l'extrémité distale et l'extrémité proximale, et permettant le passage d'un file-guide (8), ladite gaine (15) se prolongeant dans l'axe de la pointe atraumatique (3) de façon à assurer l'étanchéité avec le mandrin et à laisser libre le passage du fil guide.

## Claims

1. Device for positioning a self-expanding endoprosthesis extending between a proximal end and a distal end and comprising:
• an outer sheath (1) capable of maintaining an endoprosthesis (21) in radially contracted state,
• a core (4), which extends axially inside the outer sheath (1), is axially movable in relation to the outer sheath and at its distal end (3) has a chamber (2), which can receive an endoprosthesis (21) in radially contracted state, said chamber being delineated on its distal end by an atraumatic point (6) capable of blocking off said outer sheath, wherein the atraumatic point (6) is made of an elastomer material and has an inflatable chamber (10), and a slot (9, 9A, 9B) extending from the distal end to the proximal end of the core (4) for the passage of an inflation fluid opens into the inflatable chamber (10),
**characterised in that** the inflatable chamber (10) of the atraumatic point (6) is thin-walled and can be inflated by a pressure of less than 1 bar that is insufficient to widen a duct intended to receive the prosthesis in order to form a balloon, which immobilises the point of the core in relation to the duct.

2. Device according to Claim 1, **characterised in that** the elastomer material which the atraumatic point (6) is made of is an elastomer selected from silicon, natural rubber, polyurethane and polyether block amide.

3. Device according to any one of Claims 1 to 2, **characterised in that** the core (4) has a second slot (9'A), which extends from the distal end to the proximal end for the passage of a guide thread (8), said second slot extending into the axis of the atraumatic point.

4. Device according to any one of Claims 1 to 2, **characterised in that** the diameter of the slot (9) of the core (4) for the circulation of an inflation fluid is sufficient to simultaneously allow the passage of a guide thread (8) and the circulation of an inflation fluid.

5. Device according to Claim 4, **characterised in that** the atraumatic point (6) has an axial hole (12) for passage of a guide thread (8), the inside wall of which has at least one sealing strip (13) to ensure the seal of the contact with the guide thread (8).

6. Device according to any one of Claims 1 to 2, **characterised in that** it additionally comprises a coaxial sheath (15), which is located inside the slot (9B) of the core (4) to allow the circulation of an inflation fluid without blocking off said slot, extends from the distal end of the core and opens to the outside of the device through an opening (14) located between the distal end and the proximal end to allow the passage of a guide thread (8), said sheath (15) extending into the axis of the atraumatic point (3) in order to ensure the seal between the core and to leave the passage for the guide thread free.

## Patentansprüche

1. Vorrichtung zum Einsetzen einer selbstexpandierenden Endoprothese, die sich zwischen einem proximalen Ende und einem distalen Ende erstreckt und umfasst:
- einen äußeren Schaft (1), der in der Lage ist, eine Endoprothese (21) radial kontrahiert zu halten,
- einen Dorn (4), der sich axial im Inneren des äußeren Schaftes (1) erstreckt, in Achsenrichtung relativ zum äußeren Schaft beweglich und an seinem distalen Ende (3) eine Kammer (2), die eine radial kontrahierte Endoprothese (21) aufnehmen kann, wobei die genannte Kammer an ihrem distalen Ende in einer atraumatischen Spitze (6) endet, die den genannten äußeren Schaft verschließen kann, wobei die atraumatische Spitze (6) aus Elastomer-Werkstoff besteht und eine aufblasbare Kammer (10) aufweist, und einen Kanal (9, 9A, 9B) für den Durchtritt eines Aufblasfluids aufweist, der sich vom distalen Ende bis zum proximalen Ende des Dornes (4) erstreckt und in der aufblasbaren Kammer (10) mündet,
**dadurch gekennzeichnet, dass** die aufblasbare Kammer (10) der atraumatischen Spitze (6) dünnwandig ist und mit einem Druck von weniger als 1 bar aufgeblasen werden kann, der nicht dafür ausreicht, einen Kanal zu erweitern, der dazu bestimmt ist, die Prothese aufzunehmen, so dass ein Ballon ausgebildet wird, der die Spitze des Dorns bezüglich des Kanals immobilisiert.

2. Vorrichtung nach Patentanspruch 1, **dadurch gekennzeichnet, dass** der Elastomer-Werkstoff, aus dem die atraumatische Spitze (6) besteht, ein unter Silikon, Naturkautschuk, Polyurethan und Polyetherblockamid ausgewähltes Elastomer ist.

3. Vorrichtung nach irgendeinem der Patentansprüche 1 bis 2, **dadurch gekennzeichnet, dass** der Dorn (4) einen zweiten Kanal (9'A) für den Durchtritt eines Führungsdrahtes (8) umfasst, der sich vom distalen Ende zum proximalen Ende erstreckt, wobei sich der genannte zweite Kanal in der Achse der atraumatischen Spitze fortsetzt.

4. Vorrichtung nach irgendeinem der Patentansprüche 1 bis 2, **dadurch gekennzeichnet, dass** der Kanal (9) des Dorns (4) für den Durchfluss eines Aufblasfluids über einen ausreichenden Durchmesser verfügt, um gleichzeitig den Durchtritt eines Führungsdrahtes (8) und den Durchfluss eines Aufblasfluids zu erlauben.

5. Vorrichtung nach Patentanspruch 4, **dadurch gekennzeichnet, dass** die atraumatische Spitze (6) eine axiale Öffnung (12) für den Durchtritt eines Führungsdrahtes (8) aufweist, deren Innenwand mindestens einen Wulst (13) aufweist, um die Abdichtung der Berührung mit dem Führungsdraht (8) sicherzustellen.

6. Vorrichtung nach irgendeinem der Patentansprüche 1 bis 2, **dadurch gekennzeichnet, dass** sie außerdem einen koaxialen Schaft (15) aufweist, der im inneren des Kanals (9B) des Dorns (4) angeordnet ist und den Durchfluss eines Aufblasfluids erlaubt, ohne den genannten Kanal zu verstopfen, der sich vom distalen Ende des Dorns aus erstreckt und in einer Öffnung (14) nach außerhalb der Vorrichtung mündet, die sich zwischen dem distalen Ende und dem proximalen Ende befindet und den Durchtritt eines Führungsdrahtes (8) erlaubt, wobei sich der genannte Schaft (15) in der Achse der atraumatischen Spitze (3) derart fortsetzt, dass die Abdichtung mit dem Dorn sichergestellt und der Durchlass des Führungsdrahtes freigehalten wird.
